# EUROPEAN PATENT APPLICATION

(11) **EP 4 233 897 A1**
(43) Date of publication of application: **30.08.2023**
(21) Application number: 21883367.1
(22) Date of filing: 22.10.2021
(51) Int. Cl.: A61K 39/12, A61K 38/19, A61P 3/04, A61K 39/00

(54) **VACCINE COMPOSITION OR KIT FOR REDUCING SIZE OR VOLUME OF TARGET TISSUE, CONTAINING GENETIC MATERIAL THAT ENCODES FOREIGN ANTIGEN**

(30) Priority: 23.10.2020 KR 20200138615
(71) Applicant: SK Bioscience Co., Ltd., Bundang-gu, Seongnam-si, Gyeonggi-do, 13494 (KR)
(72) Inventor: KIM, Eun-som, Seongnam-si, Gyeonggi-do 13494 (KR); SEO, Ki-weon, Seongnam-si, Gyeonggi-do 13494 (KR); HONG, Seung-hye, Seongnam-si, Gyeonggi-do 13494 (KR); KWON, Teawoo, Seongnam-si, Gyeonggi-do 13494 (KR); KIM, Hun, Seongnam-si, Gyeonggi-do 13494 (KR); LEE, Sujeen, Seongnam-si, Gyeonggi-do 13494 (KR)
(74) Representative: Graf von Stosch Patentanwaltsgesellschaft mbH
(86) International application number: PCT/KR2021/014976
(87) International publication number: WO 2022/086300

(57) **Abstract**

The present invention relates to a vaccine composition for reducing the size or volume of a target tissue, containing a genetic material that encodes a foreign antigen and, preferably, the composition can be provided as a vaccine composition for treating or preventing obesity. The vaccine composition can be provided as a composition for removing subcutaneous adipocytes.

## Description

### [TECHNICAL FIELD]

The present application claims the priority based on Korean Patent Application No. 10-2020-0138615 filed on October 23, 2020, and the entire contents disclosed in the description and drawings of the corresponding application are incorporated in the present application. The present invention relates to a pharmaceutical composition for reducing a size or volume of target tissue, which comprises a genetic material encoding a foreign antigen to induce an immune response, and specifically, a vaccine composition for treatment which prevents and/or treats obesity. More specifically, it relates to a composition for inducing death of cells constituting a target site by immunotherapy by an antigen gene.

### [BACKGROUND ART]

Obesity refers to a 'condition in which an excessive amount of body fat is accumulated in the body' rather than simply being overweight, and is recognized as a risk factor that increases the incidence of various chronic diseases such as metabolic diseases including diabetes, cardiovascular diseases, cancer and the like. Obesity, defined as excessive accumulation of fat, is accompanied by loss of metabolic, endocrine and immune functions of adipose tissue, and pathological remodeling of this adipose tissue is attracting attention as a pathophysiological factor of major metabolic diseases.

As a drug treatment for treatment of obesity, 'Orlistat', a lipolytic enzyme inhibitor, is used, which plays a role of discharging some of fat in the body out of the body, and side effects include diarrhea and steatorrhea, and the like. In addition, in case of drugs for treatment of obesity, not only do they have many side effects, but also they can decompose fat in an undesired site of a woman's body. At present, there is no appetite suppressant that can be safely used, and in case of severely obese patients with complications, bariatric surgery for the gastrointestinal tract may be helpful.

### [DISCLOSURE]

### [TECHNICAL PROBLEM]

In order to solve the above problem, the present invention is to provide a new method or pharmaceutical composition which can reduce a size or volume of target tissue by administering into target tissue. More specifically, the method or pharmaceutical composition can be locally administered to a desired site and kill mast cells. Another embodiment is to provide a new obesity therapeutic agent or vaccine for treatment. The vaccine composition may be applied after at least one preliminary vaccine is applied to a subject.

A problem to be solved by the present invention is to provide a new type of obesity therapeutic agent or vaccine for treatment which can solve local obesity and complete a smooth body line.

A problem to be solved by the present invention is to provide a new composition for reducing adipocytes, which kills adipocytes as an immune system attacks adipocytes expressing antigenic protein to help death of adipocytes, but does not affect adipocytes in an undesired site.

### [TECHNICAL SOLUTION]

One embodiment of the present invention provides a new concept of pharmaceutical composition (preferably, vaccine composition) for reducing a size or volume of tissue, in which a genetic material encoding an antigen derived from outside the human body is administered into target tissue, and when an antigen gene is expressed in cells constituting the target tissue by the administered genetic material, against it, pre-existing immunity acts and kills the corresponding cells, and more specifically, it is to provide a vaccine for preventing or treating obesity or a composition for removing subcutaneous adipocytes.

One embodiment of the present invention provides a new concept of method for reducing a size or volume of tissue, a genetic material encoding an antigen derived from outside the human body is administered into target tissue, and when an antigen gene is expressed in cells constituting the target tissue by the administered genetic material, against it, pre-existing immunity acts and kills the corresponding cells.

The inventors of the present invention has confirmed that tissue composed by the cells can be reduced by killing cells constituting a target site using an immune response, thereby completing the present invention. In particular, it has been confirmed through an experiment that the present invention can achieve effective removal and/or reduction of adipocytes, and through this, body shape correction and obesity treatment effects can be shown.

One example of the present invention is a pharmaceutical composition to reduce the tissue by administering it into target tissue, and the composition comprises a genetic material encoding a foreign antigen. One example of the present invention provides a use of killing target cells and reducing target tissue of a genetic material encoding a foreign antigen, and more specifically, it provides a use of reducing local adipose tissue of the genetic material. Preferably, the genetic material encoding a foreign antigen comprises one or more virus selected from the group consisting of yellow fever viruses, varicella zoster viruses, and rubella viruses; or an epitope thereof. One embodiment can provide a use of killing target cells or reducing target tissue of the virus or epitope thereof. The tissue is not particularly limited, and for example, it includes tissue consisting of one or more cell selected from the group consisting of adipocytes, myocytes, osteocytes, chondrocytes, skin cells, secretory cells and hemocytes, and preferably, the tissue may be adipose tissue or muscle tissue, and more preferably, it may be subcutaneous adipose tissue. The composition may induce reduction or death of adipocytes distributed in adipocytes, particularly, subcutaneous fat.

The inventors of the present invention provides a composition, or kit which has fewer side effects than conventional liposuction, drugs, and the like, and targets only a local target site (for example, adipose tissue in a specific site), and thus can expect a body shape correction effect. The present invention provides a pharmaceutical composition for reducing a size or volume of target tissue using immunotherapy which locally acts on a target site. The present invention provides a pharmaceutical composition, vaccine composition, or vaccine composition for treatment which induces death of preferably, adipocytes, particularly, subcutaneous adipocytes.

"Target tissue or target site" used in the present description means a cell group having a structure and a function for reducing a size or volume. The "figure correction or body shape correction" means having an externally slim effect by killing or reducing cells constituting tissue (for example, adipose tissue) accumulated in an undesired site to reduce a size or volume of tissue. "Locally act" used in the present description means bring about an effect of cell death or reducing a size or volume of tissue within a radius of 10cm, 9cm, 8cm, 7cm, 6cm, 5cm, 4cm, 3cm, 2cm, 1cm, based on an injection administration site, and preferably, it may bring about the effect within a range of 3cm.

When a subject is exposed to an antigen, immunocytes functionally perform functions of phagocytosis and antigen presenting. In one embodiment, the pharmaceutical composition of the present invention may be administered after an immune environment is created by intentionally or unintentionally infiltrating a foreign substance such as a protein antigen or an epitope thereof, or the like to induce an immune response. Through another example, the pharmaceutical composition may be repeatedly administered in the body. In other example, the genetic material comprised in the pharmaceutical composition of the present invention is administered to induce pre-existing immunity, and then with repeated administration of the composition, adipocytes presenting the antigen by a substance secreted by an immune action such as a monocyte, neutrophil, natural killer cell, one kind of lymphocytes, cytotoxic T cell immunocyte, physiologically active substance, antibody, complement, or the like in the body die, and therefore, the size or volume of the adipose tissue may be reduced. The composition may be used as a use of figure correction or body shape correction, and it may be used as a use of body shape correction on a local site. In addition, the composition may be administered to muscle tissue and used as an effect of skin wrinkle improvement such as Botox, a use of figure correction through muscle contraction, and the like. Moreover, it may be used for removing benign or malignant tumors, or removing warts on skin.

The pharmaceutical composition comprises a genetic material encoding one or more antigen, and the genetic material includes not only DNA and/or RNA, but also antisense nucleotide, mRNA, ssRNA (Single-Stranded RNA), cDNA, and the like, associated with the genetic material. The antigen is a substance that causes an immune response to produce antibodies, and may include a tumor antigen, virus, bacterium, protein or epitope of the antigen. The antigen may be included without limitation as long as it can induce immunity, and the antigen is not limited thereto, but may be one or more selected from the group consisting of non-human-derived antigens, virus-derived antigens, bacterium-derived antigens, fungus-derived antigens, protozoan-derived antigens, algae-derived antigens, parasite-derived antigens, mycoplasma-derived antigens and plant-derived antigens, already well known in the art, and the antigen may be expressed as a peptide or intact protein or a part thereof. Specifically, non-limitative examples of the virus include Retroviridae (for example, human immunodeficiency virus, for example, HIV-1; Picornaviridae (for example, poliovirus, hepatitis A virus; enterovirus, human coxsackie virus, rhinovirus, echovirus); Calciviridae (for example, strain causing gastroenteritis); Togaviridae (for example, equine encephalitis virus, rubella virus); Flaviridae (for example, dengue virus, encephalitis virus, yellow fever virus); Coronoviridae (for example, corona virus); Rhabdoviridae (for example, vesicular stomatitis virus, rabies virus); Filoviridae (for example, Ebola virus); Paramyxoviridae (for example, parainfluenza virus, mumps virus, measles virus, respiratory syncytial virus); Orthomyxoviridae (for example, influenza virus); Bungaviridae (for example, Hantaan virus, Bunga virus, and phlebovirus and Nairo virus); Arena viridae (hemorrhagic fever virus); Reoviridae (for example, reovirus, orbivirus and rotavirus); Birnaviridae; Hepadnaviridae (hepatitis B virus); Parvovirida (parvovirus); Papovaviridae (papillomavirus, polyomavirus); Adenoviridae (most of adenoviruses); Herpesviridae (herpes simplex virus (HSV) 1 and 2, varicella zoster virus, cytomegalovirus (CMV), herpes virus; Poxviridae (variola virus, vaccinia virus, poxvirus); and Iridoviridae (for example, African swine fever virus). Non-limitative examples of the bacterium-derived antigen may include Pasteurella, Staphylococci, Streptococcus, Escherichia coli, Pseudomonas species, and Salmonella species. Non-limitative examples of infectious bacteria may non-limitatively include antigens derived from Helicobacter pyloris, Borelia burgdorferi, Legionella pneumophilia, Mycobacteria spp) (for example, M. tuberculosis, M. avium, M. intracellulare, M. kansaii, M. gordonae), Staphylococcus aureus, Neisseria gonorrhoeae, meningitides, Listeria monocytogenes, Streptococcus pyogenes (group A Streptococcus), Streptococcus agalactiae (group B Streptococcus), Streptococcus (viridans group), Streptococcus faecalis, Streptococcus bovis, Streptococcus (anaerobic spp), Streptococcus pneumonia, pathogenic Campylobacter sp., Enterococcus sp., Haemophilus influenzae, Bacillus antracis, Corynebacterium diphtheria, Corynebacterium spp, Erysipelothrix rhusiopathiae, Clostridium perfringers, tetani, Enterobacter aerogenes, Klebsiella pneumoniae, Pasturella multocida, Bacteroides sp., Fusobacterium nucleatum, Streptobacillus moniliformis, Treponema pallidium, pertenue, Leptospira, Rickettsia, and Actinomyces israelli. Non-limitative examples of the bacterial pathogen used in the present invention: may include Streptococcus pneumoniae, Moraxella catarrhalis, Mycoplasma pneumoniae, Klebsiella pneumoniae, Haemophilus influenza, Staphylococcus aureus, Chlamydia pneumoniae, Legionella pneumophila, or Bordetella pertusis or all bacteria which are pathogenic in lung. Non-limitative examples of the fungus-derived antigen: may be antigens derived from Aspergillus fumigatus, Blastomyces sp., Coccidioides immitis, Coccidioides posadasii, Cryptococcus neoformans, Cryptococcus gattii, Fusarium sp., Histoplasma capsulatum, Paecilomyces sp., Paracoccidiodes brasiliensis, Penicillium marneffei, Pneumocystis jirovecii, Pseudallescheria boydii, Scedosporium apiospermum, Rhizopus sp., Muco sp., Absidia sp., Cunninghamella sp., Scedosporium prolificans, Stachybotrys chartarum, Trichoderma longibrachiatum, Trichosporon sp., and the like. Parasite-derived antigenic components may be comprised, and examples thereof: may include antigens derived from cestodes (tapeworm), Taenia saginata, Taenia solium, Diphyllobothrium species, Hymenolepsis nana, Hymenolepsis diminuta, Diphylidium caninum, nematodes (ascarid), Ascaris lumbricoides, Strongyloides stercoralis, Necator americanus, Ancylostoma duodenale, Ancylostoma caninum, Trichuris trichiura, Capillaria philippinensis, Trichostrongylus species, Trichinella species, Necator americanus, Anisakis and related species, Angiostrongylus costaricensis, Enterobius vermicularis, Trematodes (trematode), Fasciolopsis buski, heterophyes species, Echinostoma species, Clonorchis sinensis, Opisthorchis species, Fasciola species, Metagonimus yokogawai, Schistosoma mansoni, Schistosoma japonicum, Schistosoma intercalatum, Echinostoma species and Paragonimus species. Preferably, it may comprise at least one virus selected from the group consisting of yellow fever viruses, varicella zoster viruses, and rubella viruses, influenza viruses, epidemic parotitis viruses and measles viruses, and preferably, it may comprise a measles virus. Preferably, the genetic material encoding the antigen may comprise a nucleic acid molecule having the nucleic acid sequence represented by SEQ ID NO: 1. Otherwise, the genetic material may comprise a nucleic acid molecule encoding a peptide having SEQ ID NO: 5 or 6.

In addition, the genetic material may comprise a polynucleotide having a nucleic acid sequence which has sequence homology of at least 85% with SEQ ID NO: 1 and can reduce a size or volume of target tissue (in other words, the present invention can achieve the object). The sequence homology may include sequence homology of 85% or more, 90% or more, 95% or more, and 99% or more. Otherwise, it may comprise a polynucleotide having a nucleic acid sequence which has sequence homology of at least 85% or more with a nucleic acid molecule encoding a peptide of SEQ ID NO: 5 or 6 and can reduce a size or volume of target tissue (in other words, the present invention can achieve the object). The sequence homology may include sequence homology of 85% or more, 90% or more, 95% or more, and 99% or more.

The composition may further comprise a genetic material encoding at least one cytokine selected from the group consisting of IL-12, IL-2, IL-4, IL-5, IFN-γ, IL-10, IL-1, IL-6, INF-alpha, INF-beta, TNF-alpha, and TNF-beta, and it is preferable to comprise IL-12 to further maximize the immunostimulatory effect by an antigen expressed by the genetic material.

The composition may be provided by comprising the genetic material in a vector delivered to an animal cell. The nucleic acid may be provided as comprised in an expression vector, for example, plasmid, and the like, and preferably, the nucleic acid may comprise transcription elements suitable for expression in a mammal cell, for example, human cell. "Vector" used in the present invention may transport a genetic material to be administered in a cell. The vector may comprise a coding sequence operably linked to an expression regulatory sequence.

In one preferable embodiment, the composition may comprise a gene corresponding to a structural protein of a yellow fever virus (17D) gene in a plasmid enabling protein expression in an animal cell (for example, gWiz^{™} vector). The yellow fever virus may be used without limitation, and for example, the yellow fever virus may include 17D strain, but not limited thereto. The plasmid may comprise a nucleic acid molecule encoding a virus or a fragment, variant or analogue thereof. For example, it may comprise SEQ ID NO: 1 or fragment, variant or analogue thereof.

In one preferable embodiment, the composition may comprise a gene corresponding to a structural protein of a rubella virus gene in a plasmid enabling protein expression in an animal cell (for example, gWiz^{™} vector). The rubella virus may be used without limitation, and for example, the rubella virus may include RA27/3 strain, but not limited thereto. The gene encoding the virus may comprise a gene encoding spike glycoproteins. The example of the spike glycoproteins may include E2-E1 heterozygotes. For example, it may comprise a nucleic acid molecule encoding protein of SEQ ID NO: 5 or a fragment, variant or analogue thereof.

In one preferable embodiment, the composition may comprise a gene corresponding to a structural protein of a varicella zoster virus gene in a plasmid enabling protein expression in an animal cell (for example, gWiz^{™} vector). The varicella zoster virus may be used without limitation, and for example, the varicella zoster virus may include Oka strain, but not limited thereto. The nucleic acid molecule encoding the virus may comprise a nucleic acid virus encoding gE protein, a surface protein. For example, it may comprise a nucleic acid molecule encoding protein of SEQ ID NO: 6 or a fragment, variant or analogue thereof.

Herein, 'fragment, variant or analogue thereof' may be understood as meaning a part of proteins, peptides, nucleic acids, nucleic acid molecules, or genes which achieve the purpose to be achieved by the proteins, peptides, nucleic acids, nucleic acid molecules, or genes of the present invention and may have sequence homology of 85% or more, 90% or more, 95% or more, or 99% or more. For example, the meaning of comprising the fragment, variant or analogue may be understood as meaning that it is not excluded from the scope of the present invention, as long as the purpose which can be achieved by the nucleic acid molecule of SEQ ID NO: 1, nucleic acid molecule encoding SEQ ID NO: 5, or nucleic acid molecule encoding SEQ ID NO: 6; or protein encoded by the nucleic acid molecule can be achieved.

The foreign genetic material of the present application may preferably include a genetic material derived from a virus, and the virus may preferably include a yellow fever virus, a rubella virus, and/or a varicella zoster virus. The virus may have an excellent effect of death of target tissue, preferably, adipocytes, and reducing a size of tissue. In addition, the effect may be quickly exhibited, and there are few side effects. Moreover, there is little risk of deterioration even if stored for a long time as an injection.

In other embodiment, the composition may further enhance an effect of adipocyte death, or reducing target tissue, by comprising an immune enhancer capable of enhancing a cellular immune response. The composition may further comprise a genetic material encoding an immune enhancer capable of improving an immune enhancing effect, for example, an auxiliary genetic material encoding interleukin 12 (IL-12). The auxiliary genetic material may be provided by inserting a coding region for example, into pSF-CMV-CMV-Sbfl vector.

The composition of the present invention may be used with not only the cytokine, a gene encoding the same or other immunoadjuvant, but also a pharmaceutical carrier or excipient as widely used in the art. The composition may be formulated for human or veterinary use, and administered in various routes. As an administration route, an oral, transdermal, intramuscular, peritoneal, intravenous, subcutaneous, or intranasal route may be used, but not limited thereto. More preferably, a transdermal, intramuscular, peritoneal or subcutaneous route may be used. In addition, the composition may be administered by any device or route in which an active material can move to a target cell, and the administration method is not particularly limited, as long as the composition of the present invention or the genetic material comprised in the composition can be delivered to target tissue or a cell constituting the tissue. For example, it may be formulated and provided as an injection, microneedle patch, or the like. For example, when used as an injection, the injection may be prepared using an aqueous solvent such as physiological saline solution, Ringer solution and the like, a non-aqueous solvent such as plant oil, higher fatty acid ester (e.g., oleic acid ethyl, etc.), alcohols (e.g., ethanol, benzyl alcohol, propylene glycol, glycerin, etc.) and the like, within a range that does not impede the effect of the genetic material, and may comprise a pharmaceutical carrier such as a stabilizer for preventing deterioration (e.g., ascorbic acid, sodium hydrogen sulfite, sodium pyrosulfite, BHA, tocopherol, EDTA, etc.), an emulsifier, a buffer for adjusting pH, a preservative for preventing microbial growth (e.g., phenyl mercury nitrate, thimerosal, benzalkonium chloride, phenol, cresol, benzyl alcohol, etc.), and the like. When used as a microneedle patch, the microneedle may include both insoluble and soluble microneedles.

In addition, the genetic material and/or auxiliary genetic material comprised in the composition may be delivered into the body according to a common gene delivery method in the art. As a non-limitative example, gene delivery methods such as physical delivery methods such as electroporation or gene gun and chemical delivery methods such as lipid-gene complex (Lipid-DNA complex: Lipoplex), polymer-gene complex (Polymer-DNA complex: Polyplex), liposome, dendrimers, nanoparticles, or other appropriate transfer vectors may all be used.

The composition of the present invention is administered in a pharmaceutically effective dose. The term "pharmaceutically effective dose" refers to an amount sufficient for a composition to exhibit an effect of treatment, improvement or prevention, or to exhibit a vaccine effect, and in addition, it means an amount sufficient to not cause side effects or a severe or excessive immune response. The composition of the present invention may be administered with a genetic material due to characteristics of the purpose of inducing an immune response and inducing cell death, and it is preferable to administer it 4-8 times, preferably, 5 times at a 2-3 day interval. When administered as described above, an effect of decrease or reduction of tissue may be obtained by cell death of the target site. The composition may have a difference in the cell death effect as it is administered in large amounts or multiple times, and may be used by adjusting an appropriate dosage for each site.

Other embodiment of the present invention provides a method for reducing or decreasing a size of volume of tissue, comprising administering a genetic material encoding a protein antigen, a vector comprising the genetic material, or a composition comprising thereof into target tissue of a subject. The method may preferably kill cells constituting subcutaneous adipose tissue. The i) method or ii) the genetic material encoding a protein antigen, a vector comprising the genetic material, or a composition comprising thereof, or a kit comprising thereof may be used as a use for improving or treating obesity of a subject, a use for wrinkle improvement, a use for muscle reduction, a use for removing or reducing benign or malignant tumors, a use for removing warts, and the like, and may be used as a use for removing fat pockets under eyes for a cosmetic purpose, and may be used as a use for alleviating dark circles through this.

The method may further comprise composing an immune environment in the body prior to administering the genetic material, a vector comprising the genetic material, or a composition comprising thereof into target issue. The composing an immune environment in the body may comprise administering a foreign substance capable of inducing an immune response. The foreign substance may include any substance as long as it uses protein as an antigen without limitation.

One embodiment of the present invention may be provided as a use for treatment, improvement or prevention of various indications, and may be provided as a cosmetic purpose for improvement of appearance, as well.

Other example of the present invention provides a kit, comprising (a) a vaccine composition for reducing a size or volume of target tissue, comprising a genetic material encoding a foreign antigen; and (b) an administration guide for administration of the vaccine. The kit may further include (c) a composition comprising a protein antigen or an epitope thereof for immune environment composition prior to administering the (a) composition. Herein, the (c) may be interpreted as at least one pre-vaccine or composition for inducing an immune response.

The `pre-vaccine' mentioned in the present application may be interpreted as a vaccine administered before the vaccine composition for reducing a size or volume of target tissue is administered, and may be understood as a vaccine composition administered before the vaccine composition for reducing a size or volume of target tissue of the present application is administered to obtain pre-existing immunity. The pre-vaccine may comprise the same antigen material as the antigen material comprised in the vaccine composition for reducing a size or volume of target tissue of the present application. For example, (a) the vaccine composition for reducing a size or volume of target tissue, comprising a genetic material encoding a foreign antigen may comprise a yellow fever virus, a rubella virus, and/or a varicella zoster virus, and (c) may also comprise a yellow fever virus, a rubella virus, and/or a varicella zoster virus.

The (a) vaccine composition may further comprise a genetic material encoding a cytokine, and any material which can be used for increasing an immune effect of the (a) composition may be comprised without limitation. The cytokine may comprise at least one selected from the group consisting of IL-12, IL-2, IL-4, IL-5, IFN-γ, IL-10, IL-1, IL-6, INF-alpha, INF-beta, TNF-alpha, and TNF-beta.

The kit of the present invention, may comprise at least one additional agent as defined in the present description in relation to a pharmaceutical composition, an antimicrobial agent, a DNase inhibitor, a RNase inhibitor, a solubilizing agent, and the like. The kit may for example, consist of at least one kit parts (for example, a container). For example, each container may be a syringe, a prefilled syringe, vial, bottle, jar, sealed sleeve, envelope or pouch, tube or blister package or any other suitable form in which the container is configured to prevent premature mixing of elements. Each of different elements may be provided individually, or some of the different elements may be provided together (i.e., in the same container). The kit may also comprise an administration guide having information on administration, administration method and dosage of any component.

Other example of the present invention provides a syringe filled with a vaccine composition for reducing a size or volume of target tissue, comprising a genetic material encoding a foreign antigen. The syringe may further comprise a genetic material encoding a cytokine. The cytokine may comprise at least one selected from the group consisting of IL-12, IL-2, IL-4, IL-5, IFN-γ, IL-10, IL-1, IL-6, INF-alpha, INF-beta, TNF-alpha, and TNF-beta.

### [ADVANTAGEOUS EFFECTS]

The present invention provides a new composition for improving or treating obesity which can kill cells on a specific target site can reduce or decrease a size or volume of tissue. By administering the composition, a body shaping effect or a body improvement effect can be obtained.

The present invention can provide a new type of obesity therapeutic agent which can solve local obesity and complete a smooth body line.

The obesity therapeutic agent of the present invention is effective for selective local site reduction, which can selectively reduce only tissue in a desired site, and does not affect tissue in an undesired site or cells constituting them.

In addition, the composition of the present invention can be used for a cosmetic purpose for appearance improvement.

### [BRIEF DESCRIPTION OF THE DRAWINGS]

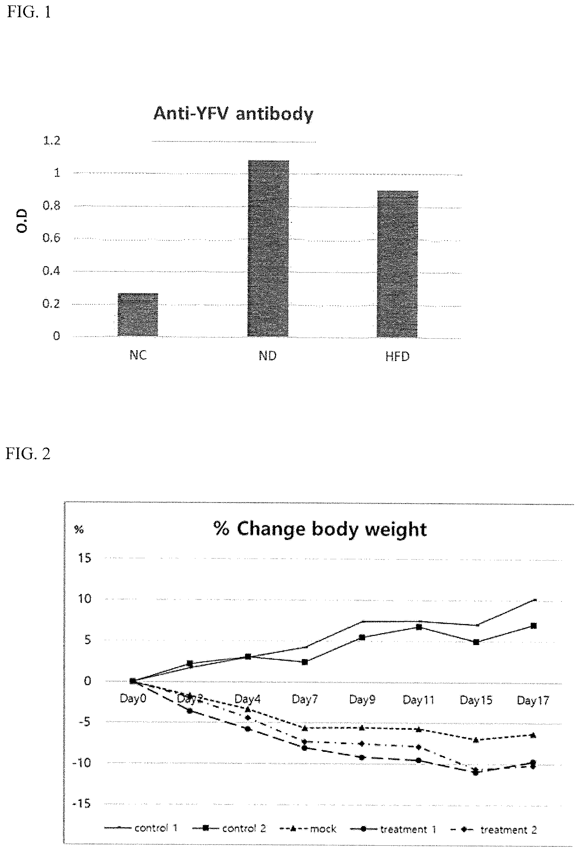

FIG. 1 shows the result of inducing pre-existing immunity by administering 17D virus, which is a yellow fever vaccine strain. This is the result of measuring the IgG antibody titer against the yellow fever virus by separating blood serum 2 weeks after administration of the vaccine strain in order to confirm composition of an immune environment. NC represents a normal mouse to which nothing is administered, and ND (Normal diet) represents a mouse fed with a normal diet, and HFD (High fat diet) represents an obesity-induced mouse in which a high calorie diet is applied, respectively.
FIG. 2 is the result of measuring the change in body weight before and after administration based on the body weight immediately before administration of the vaccine for treatment.

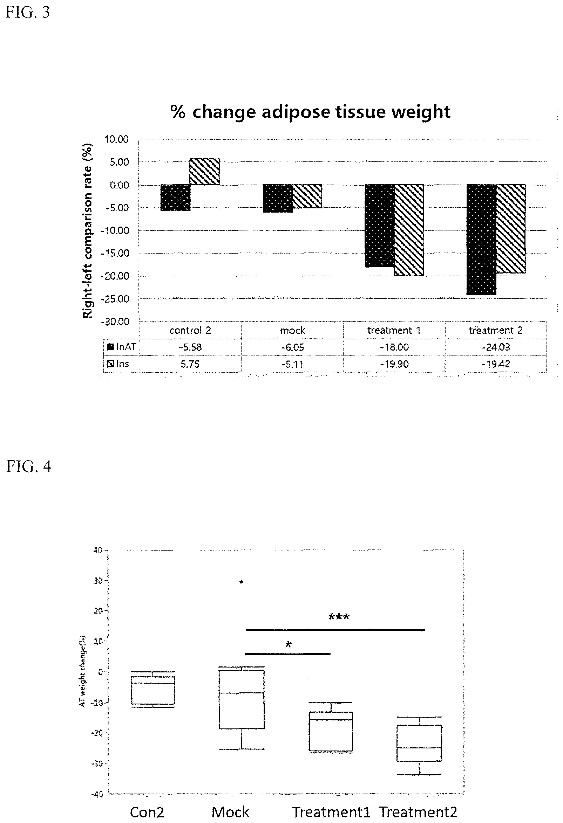

FIG. 3 is the result showing the degree of change in the weight of AT (Adipose tissue) after administration of the vaccine for treatment.
FIG. 4 shows the result of statistical analysis of the weight change rate of AT (Adipose tissue). As a result of statistical analysis, MOCK/therapeutic agent (vaccine for treatment) had a P-VALUE of 0.03007, and MOCK/therapeutic agent + IL-12 had a P-VALUE of 0.0005, and statistically, there was a significant difference in treatment of the therapeutic agent alone and therapeutic agent + IL-12, respectively, compared to MOCK. It can be seen that there was a significant difference in both groups of treatment of the therapeutic agent alone and concurrent treatment of the therapeutic agent and IL-12.

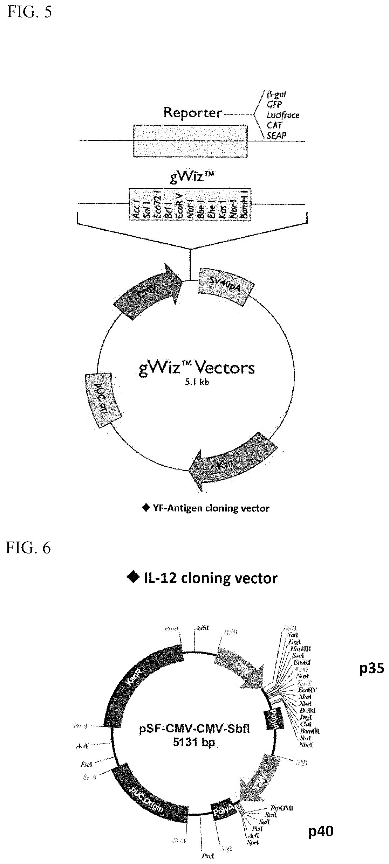

FIG. 5 shows a vector map where the genetic material (SEQ ID NO: 1) encoding structural protein (capsid protein, prM protein, envelope protein, 119 ~ 2452 bp (2334bp)) in genome (GenBank: X03700.1) of Yellow fever virus (17D) is inserted into gWiz^{™} vector. tga (stop codon) was added at the end.
FIG. 6 shows a vector map where SEQ ID NOs: 2 and 3 which are nucleic acid sequences corresponding to coding regions (p35; 127-774bp, p40; 35-1042bp) of p35 (GenBank: M86672.1) and p40 (GenBank: M86671.1) of IL-12 are inserted into pSF-CMV-CMV-Sbfl vector with a kozak sequence, respectively. IL-12 p35 was inserted into a restriction enzyme; EcoRI and XhoI sites at MCS site, and IL-12 p40 was inserted into a restriction enzyme; Sail and SpeI sites at MCS site II.

### [MODE FOR INVENTION]

Hereinafter, in order to help understanding of the present invention, it will be described in detail by examples and the like. However, the examples according to the present invention may be modified in many different forms, and the scope of the present invention should not be construed as being limited to the following examples. The examples of the present invention are provided to explain the present invention more completely to those skilled in the art.

### 1. Preparation of vaccine for degrading adipocytes

For an experiment, a genetic material for administration into a subject in which pre-existing immunity was induced and an immune enhance that could be administered together with the genetic material were prepared as follows.

### (1) Preparation of foreign antigen

### 1) Preparation of genetic material of yellow fever virus

At first, a genetic material (SEQ ID NO: 1) encoding structural protein (capsid protein, prM protein, envelope protein, 119 - 2452 bp (2334bp)) in genome (GenBank: X03700.1) of a yellow fever virus (17D) was prepared.

### 2) Preparation of genetic material of rubella virus

A genetic material encoding E1-E2 protein of RA27/3 strain of a rubella virus, encoding SEQ ID NO: 5 was prepared.

### 3) Preparation of genetic material of varicella zoster virus

A genetic material encoding gE protein of Oka strain, of a varicella zoster virus encoding SEQ ID NO: 6 was prepared.

### (2) Preparation of vector expressed in animal cell

By inserting the genetic material of the yellow fever virus into gWiz^{™} vector which could express protein in an animal cell, representatively, the adipocyte death result was confirmed. The vector map was shown in FIG. 5.

### (3) Preparation of auxiliary genetic material

Next, an immune enhancer was prepared as an auxiliary genetic material. SEQ ID NO: 2 and 3, nucleic acid sequences corresponding to coding regions (p35; 127-774bp, p40; 35-1042bp) of p35 (GenBank: M86672.1) and p40 (GenBank: M86671.1) of IL-12 were inserted into pSF-CMV-CMV-Sbfl vector with a kozak sequence, respectively. The vector map was shown in FIG. 6.

In addition, during synthesis of each IL-12, the kozak sequence was inserted at the front.

The cloned YF-antigen plasmid and mIL12-plasmid were transformed into DH5a competent cells. The corresponding cells were mass-cultured and the plasmids were recovered using a plasmid prep kit.

### 2. Confirmation of death of adipocytes

### (1) Experimental method

### 1) Diet induced obese (DIO) mouse production

C57BL/6J, 3w, female mice were fed a normal diet and a 60% high fat diet, respectively. The body weight was measured every week, and when the body weight increased by 25% or more of the normal mouse after breeding for 10-15 weeks, it was considered as an obesity-induced mouse, and used in an experiment.

### 2) Induction of pre-existing immunity and measurement of antibody titer

Pre-existing immunity formation was induced using an attenuated live virus, a vaccine strain virus. As the vaccine strain virus, 17D virus was used.

The 17D virus (attenuated live virus) was injected into the muscle of the left hind limb of the mouse at a concentration of 2 × 10⁵ pfu/time 3 times at a 2-week interval. Blood was collected 2 weeks after the last injection, and serum was separated. In the separated serum, antibodies specific to YFV were detected through ELISA analysis. Through ELISA analysis, the 17D virus used for injection was coated on a plate at a concentration of 5 × 10⁴ pfu/well, and an analysis sample (serum) was diluted and reacted for 2h. After washing, an anti-mouse IgG-HRP secondary antibody was diluted by 1/4000 and treated, and then reacted for 2h. After washing, a color forming solution was added and reacted for 10 min to measure an O.D value.

Two weeks after administering the yellow fever vaccine strain 17D virus, blood serum was separated, and the IgG antibody titer against YFV was measured, and normal immunity induction (antibody) occurred.

The result was shown in FIG. 3. In order to confirm production of antibodies in the mouse body, first, YFV-specific IgG total titer analysis was performed. The degree of titer formation was shown as absorbance (OD value) using ELISA assay. Compared to the NC group, the ND and HFD groups showed an OD value of about 1, and it could be confirmed that antibodies were produced. Therefore, it could be confirmed that immunity induction occurred.

### 3) Administration of vaccine for treatment and measurement of fat weight

Normal mice or obesity-induced mice were divided into a group administered with a yellow fever vaccine and a group not administered, respectively, and used for an experiment. All the mice were provided with a normal diet 3 days before administration of the vaccine for treatment. The vaccine for treatment was injected into inguinal adipose tissue on one side and 2 sites of interscapular adipose tissue by dividing the mice into left and right sides. IT was administered 5 times at a 2-3-day interval, and antigen DNA was at a concentration of 100ug/site, and IL-12 DNA was at a concentration of 50ug/site, and a total of 150ug/site was used, and an empty vector was used according to the total amount of antigen DNA administered. The body weight before and after administration of the vaccine for treatment was measured. On the 7th day after administration of the last vaccine for treatment, the mice were dissected, and the inguinal adipose tissue and interscapular adipose tissue were separated and each weight was measured.

ND: Normal diet, HFD: 60% High fat diet

**[Table 1]**

| Group | Obesity | Diet 1 | Diet 2 | Basal immunity (3th) | Obese vaccine (5th) | Interval | Numbe r of subject s |
|---|---|---|---|---|---|---|---|
| Control 1 | Normal | ND | ND | Medium | - | - | 5 |
| Control 2 | Normal | ND | ND | YF vaccine inoculation | - | - | 5 |
| *Mock* | DIO | HFD | ND | YF vaccine inoculation | Empty vector | 2 days | 10 |
| Vaccine for treatment 1 | | HFD | ND | YF vaccine inoculation | Antigen DNA (SEQ ID NO: 1) | 2 days | 10 |
| Vaccine for treatment 2 | | HFD | ND | YF vaccine inoculation | Antigen DNA (SEQ ID NO: 1) + IL12 DNA (SEQ ID NOs: 2 and 3) | 2 days | 10 |

The mean change rate of the body weight and the effect of reducing adipose tissue after administration of the therapeutic agent of each group of Table 1 were confirmed and shown in FIGs. 2 to 4. Referring to FIG. 2, the body weight was measured before and after administration of the vaccine for treatment (therapeutic agent, treatment), and the change in body weight was measured based on the body weight immediately before administration, and in case of the group that was not treated with anything, the body weight was gradually increased, but all the groups of Mock, vaccine for treatment 1, and vaccine for treatment 2 showed a weight loss effect, and in the administration groups of the vaccines for treatment 1 and 2, it was more significantly decreased. In addition, in case of the group co-administered with IL-12, the weight loss was shown the greatest. FIG. 3 shows the result of separating inguinal AT (adipose tissue) and interscapular AT on the left side and right side of the mice on the 7th day after 5 times of administration of the vaccine for treatment to measure the weight of AT, and comparing the amount of fat weight decrease based on an empty vector or AT not administered with the vaccine for treatment in the same subject. When compared to Control not administered with anything and the empty vector-administered group, it could be seen that the adipose tissue weight decreased when the vaccines for treatment 1 and 2 were treated. In particular, it could be seen that the change in adipose tissue weight was much greater in the treatment group of the vaccine for treatment 2 administered together with IL-12.

### [INDUSTRIAL APPLICABILITY]

The present invention can prevent and/or treat obesity. The present invention provides a method which can prevent and/or treat obesity by injecting into a localized region.

## Claims

1. A vaccine composition for reducing a size or volume of target tissue, comprising a genetic material encoding a foreign antigen.

2. The vaccine composition for reducing a size or volume of target tissue according to claim 1, wherein the target tissue is tissue consisting of one or more cell selected from the group consisting of adipocytes, myocytes, osteocytes, chondrocytes, skin cells, secretory cells and hemocytes.

3. The vaccine composition for reducing a size or volume of target tissue according to claim 1, wherein the composition induces reduction or death of adipocytes.

4. The vaccine composition for reducing a size or volume of target tissue according to claim 1, wherein the foreign antigen comprised in the composition is one or more selected from the group consisting of non-human-derived antigens, virus-derived antigens, bacterium-derived antigens, fungus-derived antigens, protozoan-derived antigens, algae-derived antigens, parasite-derived antigens, mycoplasma-derived antigens, and plant-derived antigens.

5. The vaccine composition for reducing a size or volume of target tissue according to claim 4, wherein the foreign antigen is one or more virus selected from the group consisting of yellow fever viruses, varicella zoster viruses, and rubella viruses; or an epitope thereof.

6. The vaccine composition for reducing a size or volume of target tissue according to claim 1, wherein the genetic material is DNA or RNA.

7. The vaccine composition for reducing a size or volume of target tissue according to claim 1, wherein the genetic material comprises one or more selected from the group consisting of a nucleic acid molecule of SEQ ID NO: 1; and a nucleic acid molecule encoding a peptide of SEQ ID NO: 5 or 6.

8. The vaccine composition for reducing a size or volume of target tissue according to claim 1, wherein the vaccine composition further comprises a genetic material encoding a cytokine.

9. The vaccine composition for reducing a size or volume of target tissue according to claim 8, wherein the cytokine is one or more selected from the group consisting of IL-12, IL-2, IL-4, IL-5, IFN-γ, IL-10, IL-1, IL-6, INF-alpha, INF-beta, TNF-alpha, and TNF-beta.

10. The vaccine composition for reducing a size or volume of target tissue according to claim 1, wherein the foreign antigen causes an intracellular immune response.

11. The vaccine composition for reducing a size or volume of target tissue according to claim 1, wherein the composition locally acts, and
the vaccine composition is applied after at least one protein or epitope thereof for creating an immune environment is first applied to a subject.

12. The vaccine composition for reducing a size or volume of target tissue according to claim 1, wherein the genetic material is administered to target tissue orally, by injection through transdermal, intramuscular, peritoneal, intravenous, subcutaneous or nasal route, or
by electroporation, gene gun, liposome, dendrimers, nanoparticles, or transfer vectors.

13. The vaccine composition for reducing a size or volume of target tissue according to claim 1, wherein the dose of the genetic material is 0.1~1000ug/site per one time inoculation.

14. A kit, comprising (a) a vaccine composition for reducing a size or volume of target tissue, comprising a genetic material encoding a foreign antigen; and
(b) an administration guide for administration of the vaccine.

15. The kit according to claim 14,
wherein the kit further comprise (c) a composition comprising a protein antigen or an epitope thereof for creating an immune environment prior to administration of the (a) composition.

16. The kit according to claim 14,
wherein the (a) vaccine composition further comprises a genetic material encoding a cytokine.

17. A syringe filled with a vaccine composition for reducing a size or volume of target tissue, comprising a genetic material encoding a foreign antigen.

18. The syringe according to claim 17, wherein the syringe further comprises a genetic material encoding a cytokine.

19. A composition for removing subcutaneous adipocytes comprising a genetic material encoding at least one foreign antigen.

20. The composition for removing subcutaneous adipocytes according to claim 19, wherein the composition further comprises a genetic material encoding a cytokine.

21. A method of reducing a size or volume of target tissue of a subject,
comprising administering a genetic material encoding a foreign antigen into target tissue of a subject.

22. The method according to claim 21, wherein the method induces reduction or death of adipocytes of a subject.

23. The method according to claim 21, wherein the method comprises simultaneously or sequentially introducing one or more cytokine selected from the group consisting of IL-12, IL-2, IL-4, IL-5, IFN-γ, IL-10, IL-1, IL-6, INF-alpha, INF-beta, TNF-alpha, and TNF-beta.
